# EUROPEAN PATENT APPLICATION

(11) **EP 4 180 413 A1**
(43) Date of publication of application: **17.05.2023**
(21) Application number: 21207771.3
(22) Date of filing: 11.11.2021
(51) Int. Cl.: C07C 51/09, C07C 63/26

(54) **METHOD OF CONVERTING POLY(ETHYLENE TEREPHTHALATE) TO TEREPHTHALIC ACID**

(71) Applicant: SHPP Global Technologies B.V., 4612 PX Bergen op Zoom (NL)
(72) Inventor: KENCHAIAH, Lohith, 562125, Kamataka, Bangelore (IN); KONAGHATTA NARAYANACHAR, Vinod, 562125, Kamataka, Bangelore (IN); DASH, Ranjan Kumar, Selkirk, NY 12158 (US); RAMALINGAM, Hariharan, 562125, Kamataka, Bangelore (IN); MEDIRATTA, Gaurav, 562125, Kamataka, Bangelore (IN)
(74) Representative: Bardehle Pagenberg Partnerschaft mbB Patentanwälte Rechtsanwälte

(57) **Abstract**

A method of converting poly(ethylene terephthalate) to terephthalic acid includes a glycolysis step in which poly(ethylene terephthalate) is reacted with ethylene glycol in the presence of zinc acetate catalyst, a hydrolysis step in which the product of the glycolysis step is reacted with an alkali metal hydroxide to produce an alkali metal salt of terephthalic acid, and an acidification step in which the product of the hydrolysis step is acidified to yield a precipitate containing terephthalic acid. Each step of the method can be conducted at ambient or near-ambient pressure, and the method produces terephthalic acid in high yield and high purity.

## Description

### BACKGROUND OF THE INVENTION

Poly(ethylene terephthalate) (PET) is the most commonly recycled plastic. Many processes exist to convert poly(ethylene terephthalate) to its component monomers, ethylene glycol and terephthalic acid (or terephthalic acid esters), so that they can be reused. Some methods utilize glycolysis with ethylene glycol to yield bis(hydroxyethyl terephthalate) and additional ethylene glycol. See, e.g., S. Ugduler et al., "Towards closed-loop recycling of multilayer and coloured PET plastic waste by alkaline hydrolysis," Green Chemistry, 2020, volume 22, pages 5376-5394 (Table 2, citing R. Lopez-Fonseca et al., "A kinetic study of the depolymerization of poly(ethylene terephthalate) by phase transfer catalysed alkaline hydrolysis," Journal of Chemical Technology and Biotechnology, 2009, volume 84, pages 92-99). However, such processes either fail to approach complete depolymerization of PET in a reasonable time or require high pressures and/or high temperatures to do so. Other methods utilize base-catalyzed hydrolysis to yield ethylene glycol and salts of terephthalic acid. See, e.g., J. Harvie et al., US Patent No. 5,886,057, issued 23 March 1999; and S. Ugduler et al., "Towards closed-loop recycling of multilayer and coloured PET plastic waste by alkaline hydrolysis," Green Chemistry, 2020, volume 22, pages 5376-5394. But such methods require either high pressures or organic solvents other than ethylene glycol. Still other methods utilize a combination of glycolysis and hydrolysis. See, e.g., J. Harvie et al., US Patent No. 5,886,057, issued 23 March 1999; and Czechoslovakia Patent No. CZ 299244 B6 of Sirek et al., published 28 May 2008. But these methods, too, require high pressures and/or high temperatures in the glycolysis, hydrolysis, or combined glycolysis/hydrolysis steps. There is therefore a desire for a method of depolymerizing PET to terephthalic acid in high yield, the method being conducted at temperatures less than the 260 °C melting point of poly(ethylene terephthalate) and at ambient or near-ambient pressure. Preferably, the method would also avoid the use of organic solvents other than ethylene glycol.

### BRIEF SUMMARY OF EMBODIMENTS OF THE INVENTION

One embodiment is a method of converting poly(ethylene terephthalate) to terephthalic acid, comprising: reacting poly(ethylene terephthalate) with ethylene glycol in the presence of zinc acetate at a temperature of 150 to 230 °C and an absolute pressure of 90 to 200 kilopascals for a time effective to yield a first reaction mixture comprising at least partially depolymerized poly(ethylene terephthalate); adding an alkali metal hydroxide to the first reaction mixture to yield a second reaction mixture, and maintaining the second reaction mixture at a temperature of 35 to 100 °C and a pressure of 90 to 200 kilopascals for a time effective to yield a third reaction mixture comprising ethylene glycol and an alkali metal salt of terephthalic acid; optionally, contacting the third reaction mixture with a decolorizing agent that is insoluble in the third reaction mixture to yield a fourth reaction mixture, and filtering the fourth reaction mixture to yield a filtrate; and adding an aqueous solution of an inorganic acid to the filtrate or the third reaction mixture to produce a fifth reaction mixture comprising a precipitate comprising terephthalic acid.

This and other embodiments are described in detail below.

### DETAILED DESCRIPTION OF THE INVENTION

The present inventors have determined that high yields of high purity terephthalic acid are produced by a method that includes a glycolysis step followed by a hydrolysis step and an acidification step, each step being conducted at ambient or near-ambient pressure. With the method, it is possible to avoid the use of organic solvents other than ethylene glycol.

Thus, one embodiment is a method of converting poly(ethylene terephthalate) to terephthalic acid, comprising: reacting poly(ethylene terephthalate) with ethylene glycol in the presence of zinc acetate at a temperature of 150 to 230 °C and an absolute pressure of 90 to 200 kilopascals for a time effective to yield a first reaction mixture comprising at least partially depolymerized poly(ethylene terephthalate); adding an alkali metal hydroxide to the first reaction mixture to yield a second reaction mixture, and maintaining the second reaction mixture at a temperature of 35 to 100 °C and a pressure of 90 to 200 kilopascals for a time effective to yield a third reaction mixture comprising ethylene glycol and an alkali metal salt of terephthalic acid; optionally, contacting the third reaction mixture with a decolorizing agent that is insoluble in the third reaction mixture to yield a fourth reaction mixture, and filtering the fourth reaction mixture to yield a filtrate; and adding an aqueous solution of an inorganic acid to the filtrate or the third reaction mixture to produce a fifth reaction mixture comprising a precipitate comprising terephthalic acid.

The method includes a glycolysis step, in which poly(ethylene terephthalate) is reacted with ethylene glycol in the presence of a catalyst to effect at least partial depolymerization of the poly(ethylene terephthalate). The poly(ethylene terephthalate) starting material is preferably recycled poly(ethylene terephthalate) from household and/or industrial sources. The poly(ethylene terephthalate) can be clear or colored. In some embodiments, the recycled poly(ethylene terephthalate) is rinsed with water, dilute aqueous alkali, or both prior to the glycolysis step. In some embodiments, prior to the glycolysis step the poly(ethylene terephthalate) is chipped to produce irregularly-shaped flakes having a major dimension of 0.05 to 5 centimeters, or 0.1 to 2 centimeters, as determined by optical microscopy. In a very specific embodiment of pre-treatment, the poly(ethylene terephthalate) starting material is recycled poly(ethylene terephthalate) bottles from which any separable labels have been removed, and the bottles are then water washed prior to being chipped into flakes having a major dimension of 0.05 to 5 centimeters, or 0.1 to 2 centimeters, as determined by optical microscopy, and the flakes are washed with dilute aqueous alkali then water before being used in the glycolysis step.

In the glycolysis step, poly(ethylene terephthalate) is reacted with ethylene glycol in the presence of zinc acetate as a depolymerization catalyst. In some embodiments, reacting poly(ethylene terephthalate) with ethylene glycol comprises reacting 1 to 3 moles ethylene glycol per mole of ethylene terephthalate units in the poly(ethylene terephthalate). In other words, the molar ratio of ethylene glycol to ethylene terephthalate units in the poly(ethylene terephthalate) can be 1:1 to 3:1. Within this range, the molar ratio of ethylene glycol to ethylene terephthalate units in the poly(ethylene terephthalate) can be 1.5:1 to 3:1, or 1.5:1 to 2.5:1.

In some embodiments, reacting poly(ethylene terephthalate) with ethylene glycol in the presence of zinc acetate comprises reacting 100 parts by weight poly(ethylene terephthalate) in the presence of 0.025 to 0.4 parts by weight zinc acetate. Within the range of 0.025 to 0.4 parts by weight, the zinc acetate amount can be 0.05 to 0.2 parts by weight, or 0.05 to 0.15 parts by weight, per 100 parts by weight poly(ethylene terephthalate).

The reaction of poly(ethylene terephthalate) with ethylene glycol in the presence of zinc acetate is conducted at a temperature of 150 to 230 °C. Within this range, the temperature can be 150 to 210 °C, 160 to 200 °C, or 170 to 200 °C, or 180 to 230 °C, or 180 to 200 °C, or 185 to 200 °C. The reaction is conducted at an absolute pressure of 90 to 200 kilopascals. Within this range, the absolute pressure can be 90 to 150 kilopascals, or 90 to 110 kilopascals. It is an important advantage of the present method that the glycolysis step can be conducted at ambient or near-ambient pressure. The reaction is conducted for a time effective to yield a first reaction mixture comprising at least partially depolymerized poly(ethylene terephthalate). The extent of depolymerization can be determined by analyzing the first reaction mixture by gel permeation chromatography with polystyrene standards to determine the weight average molecular weight of the partially depolymerized poly(ethylene terephthalate). In some embodiments, the at least partially depolymerized poly(ethylene terephthalate) has a weight average molecular weight of 500 to 10,000 grams/mole, determined by gel permeation chromatography using polystyrene standards. Within the range of 500 to 10,000 grams/mole, the weight average molecular weight of the at least partially depolymerized poly(ethylene terephthalate) can be 500 to 5,000 grams/mole, or 500 to 2,000 grams/mole. These values can be compared to the weight average molecular weight of the poly(ethylene terephthalate) starting material, which is typically on the order of 60,000 grams/mole, and the molecular weight of the complete glycolysis product, bis(hydroxyethyl) terephthalate, which is about 254 grams/mole.

In some embodiments, the glycolysis reaction is conducted for 1 to 6 hours, or 1.5 to 6 hours, or 2 to 5 hours. In a very specific embodiment of the glycolysis step, reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours.

In some embodiments, the glycolysis step comprises little or no hydrolysis. Specifically, in these embodiments, reacting poly(ethylene terephthalate) with ethylene glycol in the presence of zinc acetate is conducted in the presence of 0 to 1 parts by weight water, based on 100 parts by weight of the poly(ethylene terephthalate). Within the range of 0 to 1 parts by weight, the water amount can be 0 to 0.5 parts by weight, or 0 to 0.1 parts by weight, or zero parts by weight per 100 parts by weight of the poly(ethylene terephthalate).

In addition to the glycolysis step, the method includes a hydrolysis step that follows the glycolysis step. The hydrolysis step can immediately follow the glycolysis step, or the hydrolysis step can follow one or more intermediate steps between the glycolysis step and the hydrolysis step. In the hydrolysis step, an alkali metal hydroxide is added to the first reaction mixture to yield a second reaction mixture, and the second reaction mixture is maintained at a temperature of 35 to 100 °C and an absolute pressure of 90 to 200 kilopascals for a time effective to yield a third reaction mixture comprising ethylene glycol and an alkali metal salt of terephthalic acid. Within the range of 35 to 100 °C, the temperature of the hydrolysis step can be 35 to 90 °C, or 35 to 70 °C, or 35 to 60 °C, or 40 to 90 °C, or 40 to 70 °C, or 40 to 60 °C. Within the range of 90 to 200 kilopascals, the absolute pressure of the hydrolysis step can be 90 to 150 kilopascals, or 90 to 110 kilopascals. Suitable alkali metal hydroxides include lithium hydroxide, sodium hydroxide, potassium hydroxide, and combinations thereof. In some embodiments, the alkali metal hydroxide comprises sodium hydroxide. In some embodiments, adding the alkali metal hydroxide to the first reaction mixture comprises adding 2 to 5 moles of the alkali metal hydroxide, based on 1 mole of ethylene terephthalate units in the poly(ethylene terephthalate). Within this range, the moles of alkali metal hydroxide can be 2 to 4 moles, or 2.5 to 3.5 moles, based on 1 mole of ethylene terephthalate units in the poly(ethylene terephthalate). The hydrolysis step is conducted for a time effective to yield a third reaction mixture comprising ethylene glycol and an alkali metal salt of terephthalic acid. In some embodiments, hydrolysis step reaction time is 0.25 to 4 hours, or 0.5 to 2 hours, or 0.5 to 1.5 hours. In a very specific embodiment of the hydrolysis step, the second reaction mixture is maintained at a temperature of 35 to 60 °C and a pressure of 90 to 110 kilopascals for a time of 0.5 to 2 hours to yield the third reaction mixture.

The method optionally comprises a decolorizing step, which is particularly useful when the poly(ethylene terephthalate) starting material includes colored material (e.g., colored poly(ethylene terephthalate) bottles). The decolorizing step comprises contacting the third reaction mixture with a decolorizing agent that is insoluble in the third reaction mixture to yield a fourth reaction mixture, and filtering the fourth reaction mixture to yield a filtrate. Suitable decolorizing agents include activated carbon, alumina, silica, and combinations thereof. In some embodiments, the decolorizing agent comprises activated carbon. In some embodiments, the decolorizing agent can be used in an amount of 1 to 100 parts by weight per 100 parts by weight poly(ethylene terephthalate). Within this range, the decolorizing agent amount can be 2 to 50 parts by weight, or 5 to 20 parts by weight, per 100 parts by weight poly(ethylene terephthalate). In a very specific embodiment of the decolorizing step, the second reaction mixture is contacted with 5 to 20 parts by weight activated carbon per 100 parts by weight poly(ethylene terephthalate).

In addition to the glycolysis step, the hydrolysis step, and the optional decolorizing step, the method comprises an acidification step. In the acidification step, an aqueous solution of an inorganic acid is added to the filtrate (if the decolorizing step was conducted) or to the third reaction mixture (if the decolorizing step was not conducted) to produce a fifth reaction mixture comprising a precipitate comprising terephthalic acid. Suitable inorganic acids include hydrochloric acid, sulfuric acid, and combinations thereof. In some embodiments, the inorganic acid comprises hydrochloric acid. The amount of inorganic acid used is an amount effective to reduce the pH of the solution to which it is added to a value less than or equal to 2 (the pKa values of terephthalic acid are approximately 3.5 and 4.8). Within this limit, the amount of inorganic acid used can be an amount effective to reduce the pH of the solution to which it is added to a value of 1 to 2, or a value of 1.5 to 2.

The method can, optionally, further comprise isolating the precipitate from the fifth reaction mixture. In some embodiments, the method further comprises filtering the fifth reaction mixture to separate the precipitate, washing the separated precipitate, and drying the washed and separated precipitate. As demonstrated in the working examples below, terephthalic acid yields of greater than 90 mole percent, and in some cases greater than 95 mole percent can be achieved, wherein mole percent values are based on moles of ethylene terephthalate units in the poly(ethylene terephthalate) starting material. In some embodiments, the yield of isolated terephthalic acid is 94 to 97.5 mole percent, based on moles of ethylene terephthalate units in the poly(ethylene terephthalate) starting material. And the purity of the isolated terephthalic acid can be greater than 99.5 percent, based on 100 percent total peak area in a 254 nanometer absorbance chromatogram derived from high performance liquid chromatography. In some embodiments, the purity of the isolated terephthalic acid is 95 to 99.7 percent, or 98 to 99.7 percent, or 99 to 99.7 percent, based on 100 percent total peak area in a 254 nanometer absorbance chromatogram derived from high performance liquid chromatography. The only significant impurity in the isolated terephthalic acid is isophthalic acid, which is typically present as in small amounts as a comonomer with terephthalic acid in the poly(ethylene terephthalate) starting material. The isophthalic acid is typically present in an amount of less than or equal to 0.5 percent, based on 100 percent total peak area in a 254 nanometer absorbance chromatogram derived from high performance liquid chromatography.

In some embodiments of the method, ethylene glycol is separated from the first reaction mixture before an alkali metal hydroxide is added to the first reaction mixture. In some embodiments of the method, ethylene glycol is not separated from the first reaction mixture before an alkali metal hydroxide is added to the first reaction mixture. In still other embodiments, ethylene glycol is separated from the filtrate (if a decolorizing step is conducted) or the fifth reaction mixture (if a decolorizing step is not conducted).

In some embodiments, the method excludes a transesterification reaction between the glycolysis step and the hydrolysis step. In some embodiments, the method does not employ an organic solvent other than ethylene glycol (e.g., it does not employ ethanol). In some embodiments, the method does not employ an aromatic hydrocarbon solvent.

In a very specific embodiment of the method, the reacting poly(ethylene terephthalate) with ethylene glycol comprises reacting 100 parts by weight poly(ethylene terephthalate) with 50 to 80 parts by weight ethylene glycol; the reacting poly(ethylene terephthalate) with ethylene glycol in the presence of zinc acetate comprises reacting 100 parts by weight poly(ethylene terephthalate) in the presence of 0.05 to 0.2 parts by weight zinc acetate; the reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours; the reacting poly(ethylene terephthalate) with ethylene glycol in the presence of zinc acetate is conducted in the presence of 0 to 0.1 parts by weight water, based on 100 parts by weight of the poly(ethylene terephthalate); the alkali metal hydroxide comprises sodium hydroxide; the adding an alkali metal hydroxide to the first reaction mixture comprises adding 1.5 to 5 moles of the alkali metal hydroxide, based on 1 mole of ethylene terephthalate units in the poly(ethylene terephthalate); the second reaction mixture is maintained at a temperature of 35 to 60 °C and a pressure of 90 to 110 kilopascals, for a time of 0.5 to 2 hours to yield the third reaction mixture; the method comprises the contacting the second reaction mixture with a decolorizing agent, and the contacting the second reaction mixture with a decolorizing agent comprises contacting the second reaction mixture with 5 to 20 parts by weight activated carbon per 100 parts by weight poly(ethylene terephthalate); the inorganic acid comprises hydrochloric acid; and the method further comprises isolating the precipitate from the fifth reaction mixture.

In some embodiments, the method further comprises isolating the precipitate from the fifth reaction mixture, and using the isolated precipitate in a reaction to form a product selected from the group consisting of dimethyl terephthalate, dioctyl terephthalate, bis(hydroxyethyl) terephthalate, poly(ethylene terephthalate), glycol-modified poly(ethylene terephthalate), poly(butylene terephthalate), poly(butylene adipate-co-terephthalate), poly(trimethylene terephthalate), poly(cyclohexylenedimethylene terephthalate), glycol-modified poly(cyclohexylenedimethylene terephthalate), poly(cyclohexylenedimethylene terephthalate-co-isophthalate), poly(hexamethylene terephthalamide), poly(ethylene terephthalamide), poly(butylene terephthalamide), poly(2-methylpentamethylene terephthalamide), and poly(*para*-phenylene terephthalamide).

All ranges disclosed herein are inclusive of the endpoints, and the endpoints are independently combinable with each other. Each range disclosed herein constitutes a disclosure of any point or sub-range lying within the disclosed range.

The invention includes at least the following aspects.
Aspect 1: A method of converting poly(ethylene terephthalate) to terephthalic acid, comprising: reacting poly(ethylene terephthalate) with ethylene glycol in the presence of zinc acetate at a temperature of 150 to 230 °C and an absolute pressure of 90 to 200 kilopascals for a time effective to yield a first reaction mixture comprising at least partially depolymerized poly(ethylene terephthalate); adding an alkali metal hydroxide to the first reaction mixture to yield a second reaction mixture, and maintaining the second reaction mixture at a temperature of 35 to 100°C and an absolute pressure of 90 to 200 kilopascals for a time effective to yield a third reaction mixture comprising ethylene glycol and an alkali metal salt of terephthalic acid; optionally, contacting the third reaction mixture with a decolorizing agent that is insoluble in the third reaction mixture to yield a fourth reaction mixture, and filtering the fourth reaction mixture to yield a filtrate; and adding an aqueous solution of an inorganic acid to the filtrate or the third reaction mixture to produce a fifth reaction mixture comprising a precipitate comprising terephthalic acid.
Aspect 2: The method of aspect 1, wherein the reacting poly(ethylene terephthalate) with ethylene glycol comprises reacting 1 to 3 moles ethylene glycol per mole of ethylene terephthalate units in the poly(ethylene terephthalate).
Aspect 3: The method of aspect 1 or 2, wherein the reacting poly(ethylene terephthalate) with ethylene glycol in the presence of zinc acetate comprises reacting 100 parts by weight poly(ethylene terephthalate) in the presence of 0.025 to 0.4 parts by weight zinc acetate.
Aspect 4: The method of any one of aspects 1-3, wherein the reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours.
Aspect 5: The method of any one of aspects 1-4, wherein the at least partially depolymerized poly(ethylene terephthalate) has a weight average molecular weight of 500 to 10,000 grams/mole, determined by gel permeation chromatography using polystyrene standards.
Aspect 6: The method of any one of aspects 1-5, wherein the reacting poly(ethylene terephthalate) with ethylene glycol in the presence of zinc acetate is conducted in the presence of 0 to 1 parts by weight water, based on 100 parts by weight of the poly(ethylene terephthalate).
Aspect 7: The method of any one of aspects 1-6, wherein the alkali metal hydroxide comprises sodium hydroxide.
Aspect 8: The method of any one of aspects 1-7, wherein the adding an alkali metal hydroxide to the first reaction mixture comprises adding 2 to 5 moles of the alkali metal hydroxide, based on 1 mole of ethylene terephthalate units in the poly(ethylene terephthalate).
Aspect 9: The method of any one of aspects 1-8, wherein the second reaction mixture is maintained at a temperature of 35 to 60 °C and a pressure of 90 to 110 kilopascals, for a time of 0.5 to 2 hours to yield the third reaction mixture.
Aspect 10: The method of any one of aspects 1-9, comprising the contacting the second reaction mixture with a decolorizing agent.
Aspect 11: The method of aspect 10, wherein the contacting the second reaction mixture with a decolorizing agent comprises contacting the second reaction mixture with 5 to 20 parts by weight activated carbon per 100 parts by weight poly(ethylene terephthalate).
Aspect 12: The method of any one of aspects 1-11, wherein the inorganic acid comprises hydrochloric acid.
Aspect 13: The method of any one of aspects 1-12, further isolating the precipitate from the fifth reaction mixture.
Aspect 14: The method of aspect 1, wherein the reacting poly(ethylene terephthalate) with ethylene glycol comprises reacting 100 parts by weight poly(ethylene terephthalate) with 50 to 80 parts by weight ethylene glycol; the reacting poly(ethylene terephthalate) with ethylene glycol in the presence of zinc acetate comprises reacting 100 parts by weight poly(ethylene terephthalate) in the presence of 0.05 to 0.2 parts by weight zinc acetate; the reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours; the reacting poly(ethylene terephthalate) with ethylene glycol in the presence of zinc acetate is conducted in the presence of 0 to 0.1 parts by weight water, based on 100 parts by weight of the poly(ethylene terephthalate); the alkali metal hydroxide comprises sodium hydroxide; the adding an alkali metal hydroxide to the first reaction mixture comprises adding 1.5 to 5 moles of the alkali metal hydroxide, based on 1 mole of ethylene terephthalate units in the poly(ethylene terephthalate); the second reaction mixture is maintained at a temperature of 35 to 60 °C and a pressure of 90 to 110 kilopascals, for a time of 0.5 to 2 hours to yield the third reaction mixture; the method comprises the contacting the second reaction mixture with a decolorizing agent, and the contacting the second reaction mixture with a decolorizing agent comprises contacting the second reaction mixture with 5 to 20 parts by weight activated carbon per 100 parts by weight poly(ethylene terephthalate); the inorganic acid comprises hydrochloric acid; and the method further comprises isolating the precipitate from the fifth reaction mixture.
Aspect 15: The method of aspect 13 or 14, further comprising using the isolated precipitate in a reaction to form a product selected from the group consisting of dimethyl terephthalate, dioctyl terephthalate, bis(hydroxyethyl) terephthalate, poly(ethylene terephthalate), glycol-modified poly(ethylene terephthalate), poly(butylene terephthalate), poly(butylene adipate-co-terephthalate), poly(trimethylene terephthalate), poly(cyclohexylenedimethylene terephthalate), glycol-modified poly(cyclohexylenedimethylene terephthalate), poly(cyclohexylenedimethylene terephthalate-co-isophthalate), poly(hexamethylene terephthalamide), poly(ethylene terephthalamide), poly(butylene terephthalamide), poly(2-methylpentamethylene terephthalamide), and poly(*para*-phenylene terephthalamide).

The invention is further illustrated by the following non-limiting examples.

### EXAMPLES

Materials used in these examples are summarized in Table 1. Poly(ethylene terephthalate) (PET) was provided in the form of flakes derived from a process that started with recycled PET bottles and included removing the necks and printed labels from the bottles, water washing the remaining bottle parts, chipping the bottle parts into irregularly-shaped flakes of having a major dimension of about 5 millimeters, washing the flakes with dilute alkali followed by hot water, and drying the flakes.

**Table 1**

| Material | Description |
|---|---|
| PET | Poly(ethylene terephthalate), CAS Reg. No. 25038-59-9, in the form of flakes derived from waste poly(ethylene terephthalate) bottles; based on gel permeation chromatography with polystyrene standards, weight average molecular weight = 58,226 grams/mole, and number average molecular weight = 26,268 grams/mole. |
| EG | Ethylene glycol, CAS Reg. No. 107-21-1. |
| Zn(OAc)₂ | Zinc acetate dihydrate, CAS Reg. No. 5970-45-6. |
| NaOH | Sodium hydroxide, CAS Reg. No. 1310-73-2. |
| Carbon | Activated carbon, CAS Reg. No. 7440-44-0, obtained from Sigma-Aldrich. |

Reaction conditions for five inventive examples are summarized in Table 2, where "PET (g)" is the weight of PET flakes in grams; "PET (moles)" is the moles of terephthalic acid calculated based on the weight of PET flakes; "EG (g)" is the weight of ethylene glycol, in grams, used in the glycolysis step; "EG (equiv.)" is the moles of ethylene glycol per mole of ethylene terephthalate units linkages in the PET; "Zn(OAc)₂ (g)" is the weight of zinc acetate catalyst, in grams; "Zn(OAc)₂ (equiv.)" is the equivalents of zinc acetate per mole of terephthalic acid in the PET; "NaOH (g)" is the weight of sodium hydroxide used in the hydrolysis step, in grams; "NaOH (equiv.)" is the equivalents of sodium hydroxide, based on equivalents of ester linkages in the PET; "Demineralized water (g)" is the weight of demineralized water, in grams, combined with the sodium hydroxide before addition to the glycolysis reaction product; "Activated Carbon (g)" is the weight of activated carbon, in grams, added to the hydrolysis reaction product; "Glycolysis reaction time (h)" is the glycolysis time in hours; "Hydrolysis reaction time (h)" is the hydrolysis time in hours; "TPA content of product (%)" is the content of terephthalic acid based on 100 percent total peak area in the 254 nanometer absorbance chromatogram from high performance liquid chromatographic analysis of the isolated solid; "IPA content of product (%)" is the content of isophthalic acid based on 100 percent total peak area in the 254 nanometer absorbance chromatogram from high performance liquid chromatographic analysis of the isolated solid.

The procedure for Example 3 follows. PET flakes (100 grams) were combined with ethylene glycol (EG; 65 grams) in a three neck round bottom flask, equipped with overhead stirrer and water cooled condenser. Zinc acetate catalyst (0.1 gram) was added to the flask, and the resulting mixture was heated to 190 °C and maintained at that temperature for 3 hours. The purpose of this glycolysis step was to convert insoluble PET flakes to soluble oligomers that could be more easily hydrolyzed in the subsequent hydrolysis step. After 3 hours, the reaction mixture was cooled to 50 °C with stirring, then 54 grams of a 2 weight percent sodium hydroxide solution was added to the flask, and stirring was continued for one hour at 50 °C. This hydrolysis step converted the PET oligomers to ethylene glycol and terephthalic acid disodium salt. Activated carbon (10 grams) was then added to the flask and the resulting mixture was stirred for 1 hour at 50 °C, after which the mixture was filtered to remove the activated carbon. Aqueous hydrochloric acid (20 weight percent) was then added to the filtrate until the pH was less than 2, resulting in the formation of a precipitate. The precipitate was filtered, washed with about 750 grams of water, dried at room temperature while pulling air through the filtrate, then dried in a vacuum oven at 100 °C for ten hours. HPLC analysis of the resulting solid indicated that it was 99.55 percent terephthalic acid and 0.43 weight percent isophthalic acid, based on 100 percent total peak area in the 254 nanometer absorbance chromatogram. The yield was 84.0 grams, corresponding to 97.19 percent of the theoretical yield based on the weight of PET.

The reaction mixture obtained after glycolysis but before hydrolysis was subjected to analysis by gel permeation chromatography (GPC) with polystyrene standards, and liquid chromatography/mass spectrometry (LC/MS). GPC indicated that the partially depolymerized poly(ethylene terephthalate) in the reaction mixture had a weight average molecular weight of 980 grams/mole and a number average molecular weight of 604 grams/mole. LC/MS indicated that the major components of the partially depolymerized poly(ethylene terephthalate) were bis(hydroxyethyl) terephthalate (BHET; exact mass = 254.08 grams/mole), 1,2-bis(2-hydroxyethoxyterephthaloyloxy)ethane (exact mass = 446.12 grams/mole), and bis(2-hydroxyethoxyterephthaloyloxyethyl) terephthalate (exact mass = 638.16 grams/mole).

Variations in reaction conditions and product analyses for Examples 1-5 are summarized in Table 2.

**Table 2**

| | Ex. 1 | Ex. 2 | Ex. 3 | Ex. 4 | Ex. 5 |
|---|---|---|---|---|---|
| PET (g) | 25 | 50 | 100 | 50 | 50 |
| PET (moles) | 0.13 | 0.26 | 0.52 | 0.25 | 0.26 |
| EG (g) | 16.13 | 32.26 | 64.52 | 32.26 | 32.26 |
| EG (equiv.) | 2.0 | 2.0 | 2.0 | 2.0 | 2.0 |
| Zn(OAc)₂ (g) | 0.025 | 0.05 | 0.10 | 0.05 | 0.05 |
| Zn(OAc)₂ (equiv.) | 0.0008 | 0.0008 | 0.0008 | 0.0008 | 0.0008 |
| NaOH (g) | 13.52 | 27.00 | 54.00 | 27.00 | 27.00 |
| NaOH (equiv.) | 2.61 | 2.60 | 2.60 | 2.60 | 2.60 |
| Demineralized water (g) | 250 | 500 | 1000 | 400 | 400 |
| Activated Carbon (g) | 2.5 | 5.0 | 10.0 | 2.5 | 2.5 |
| Glycolysis reaction time (h) | 3.0 | 3.0 | 3.0 | 3.0 | 3.0 |
| Hydrolysis reaction time (h) | 1.0 | 1.0 | 1.0 | 1.0 | 1.0 |
| TPA content of product (%) | 99.49 | 99.56 | 99.55 | 99.55 | 99.58 |
| IPA content of product (%) | 0.43 | 0.41 | 0.43 | 0.44 | 0.41 |
| Yield (g) | 19.0 | 41.0 | 84.0 | 42.0 | 42.0 |
| Yield (%) | 87.96 | 94.89 | 97.19 | 97.19 | 97.19 |

The results in Table 2 show that a high yield of high purity terephthalic acid is produced by the present method, which includes a glycolysis step followed by a hydrolysis step, each conducted at ambient or near-ambient pressure and a temperature below the melting point of the poly(ethylene terephthalate) starting material.

## Claims

1. A method of converting poly(ethylene terephthalate) to terephthalic acid, comprising:
reacting poly(ethylene terephthalate) with ethylene glycol in the presence of zinc acetate at a temperature of 150 to 230 °C and an absolute pressure of 90 to 200 kilopascals for a time effective to yield a first reaction mixture comprising at least partially depolymerized poly(ethylene terephthalate);
adding an alkali metal hydroxide to the first reaction mixture to yield a second reaction mixture, and maintaining the second reaction mixture at a temperature of 35 to 100°C and an absolute pressure of 90 to 200 kilopascals for a time effective to yield a third reaction mixture comprising ethylene glycol and an alkali metal salt of terephthalic acid;
optionally, contacting the third reaction mixture with a decolorizing agent that is insoluble in the third reaction mixture to yield a fourth reaction mixture, and filtering the fourth reaction mixture to yield a filtrate; and
adding an aqueous solution of an inorganic acid to the filtrate or the third reaction mixture to produce a fifth reaction mixture comprising a precipitate comprising terephthalic acid.

2. The method of claim 1, wherein the reacting poly(ethylene terephthalate) with ethylene glycol comprises reacting 1 to 3 moles ethylene glycol per mole of ethylene terephthalate units in the poly(ethylene terephthalate).

3. The method of claim 1 or 2, wherein the reacting poly(ethylene terephthalate) with ethylene glycol in the presence of zinc acetate comprises reacting 100 parts by weight poly(ethylene terephthalate) in the presence of 0.025 to 0.4 parts by weight zinc acetate.

4. The method of any one of claims 1-3, wherein the reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours.

5. The method of any one of claims 1-4, wherein the at least partially depolymerized poly(ethylene terephthalate) has a weight average molecular weight of 500 to 10,000 grams/mole, determined by gel permeation chromatography using polystyrene standards.

6. The method of any one of claims 1-5, wherein the reacting poly(ethylene terephthalate) with ethylene glycol in the presence of zinc acetate is conducted in the presence of 0 to 1 parts by weight water, based on 100 parts by weight of the poly(ethylene terephthalate).

7. The method of any one of claims 1-6, wherein the alkali metal hydroxide comprises sodium hydroxide.

8. The method of any one of claims 1-7, wherein the adding an alkali metal hydroxide to the first reaction mixture comprises adding 2 to 5 moles of the alkali metal hydroxide, based on 1 mole of ethylene terephthalate units in the poly(ethylene terephthalate).

9. The method of any one of claims 1-8, wherein the second reaction mixture is maintained at a temperature of 35 to 60 °C and a pressure of 90 to 110 kilopascals, for a time of 0.5 to 2 hours to yield the third reaction mixture.

10. The method of any one of claims 1-9, comprising the contacting the second reaction mixture with a decolorizing agent.

11. The method of claim 10, wherein the contacting the second reaction mixture with a decolorizing agent comprises contacting the second reaction mixture with 5 to 20 parts by weight activated carbon per 100 parts by weight poly(ethylene terephthalate).

12. The method of any one of claims 1-11, wherein the inorganic acid comprises hydrochloric acid.

13. The method of any one of claims 1-12, further isolating the precipitate from the fifth reaction mixture.

14. The method of claim 1, wherein
the reacting poly(ethylene terephthalate) with ethylene glycol comprises reacting 100 parts by weight poly(ethylene terephthalate) with 50 to 80 parts by weight ethylene glycol;
the reacting poly(ethylene terephthalate) with ethylene glycol in the presence of zinc acetate comprises reacting 100 parts by weight poly(ethylene terephthalate) in the presence of 0.05 to 0.2 parts by weight zinc acetate;
the reacting poly(ethylene terephthalate) with ethylene glycol in the presence of zinc acetate is conducted at a temperature of 180 to 230 °C and an absolute pressure of 90 to 110 kilopascals, for a time of 1.5 to 6 hours;
the reacting poly(ethylene terephthalate) with ethylene glycol in the presence of zinc acetate is conducted in the presence of 0 to 0.1 parts by weight water, based on 100 parts by weight of the poly(ethylene terephthalate);
the alkali metal hydroxide comprises sodium hydroxide;
the adding an alkali metal hydroxide to the first reaction mixture comprises adding 1.5 to 5 moles of the alkali metal hydroxide, based on 1 mole of ethylene terephthalate units in the poly(ethylene terephthalate);
the second reaction mixture is maintained at a temperature of 35 to 60 °C and a pressure of 90 to 110 kilopascals, for a time of 0.5 to 2 hours to yield the third reaction mixture;
the method comprises the contacting the second reaction mixture with a decolorizing agent, and the contacting the second reaction mixture with a decolorizing agent comprises contacting the second reaction mixture with 5 to 20 parts by weight activated carbon per 100 parts by weight poly(ethylene terephthalate);
the inorganic acid comprises hydrochloric acid; and
the method further comprises isolating the precipitate from the fifth reaction mixture.

15. The method of claim 13 or 14, further comprising using the isolated precipitate in a reaction to form a product selected from the group consisting of dimethyl terephthalate, dioctyl terephthalate, bis(hydroxyethyl) terephthalate, poly(ethylene terephthalate), glycol-modified poly(ethylene terephthalate), poly(butylene terephthalate), poly(butylene adipate-co-terephthalate), poly(trimethylene terephthalate), poly(cyclohexylenedimethylene terephthalate), glycol-modified poly(cyclohexylenedimethylene terephthalate), poly(cyclohexylenedimethylene terephthalate-co-isophthalate), poly(hexamethylene terephthalamide), poly(ethylene terephthalamide), poly(butylene terephthalamide), poly(2-methylpentamethylene terephthalamide), and poly(*para*-phenylene terephthalamide).
